# EUROPEAN PATENT APPLICATION

(11) **EP 3 476 404 A1**
(43) Date of publication of application: **01.05.2019**
(21) Application number: 17815045.4
(22) Date of filing: 11.05.2017
(51) Int. Cl.: A61L 2/07, F17C 5/02

(54) **GERM-FREE LIQUEFIED GAS PRODUCTION APPARATUS**

(30) Priority: 24.06.2016 JP 2016125997
(71) Applicant: Ulvac, Inc., Chigasaki-shi Kanagawa 253-8543 (JP); Ulvac Cryogenics Incorporated, Chigasaki-shi, Kanagawa 253-0085 (JP)
(72) Inventor: YOSHIMOTO Tsuyoshi, Chigasaki-shi Kanagawa 253-8543 (JP); OHINATA Yoichi, Chigasaki-shi Kanagawa 253-8543 (JP); KINOSHITA Takao, Chigasaki-shi Kanagawa 253-8543 (JP); FURUYA Shinji, Chigasaki-shi Kanagawa 253-0085 (JP); SUZUKI Naoto, Chigasaki-shi Kanagawa 253-0085 (JP); HIRUMA Masayoshi, Chigasaki-shi Kanagawa 253-0085 (JP); TAKASAKI Shinji, Chigasaki-shi Kanagawa 253-0085 (JP)
(74) Representative: Zacco GmbH
(86) International application number: PCT/JP2017/017835
(87) International publication number: WO 2017/221575

(57) **Abstract**

The present invention provides a sterilized-liquefied gas producing apparatus including: a liquefied gas reservoir; a source-gas supplier that supplies a source gas to the liquefied gas reservoir; a cooler that cools down an inside of the liquefied gas reservoir to liquefy the source gas; a supply pipe that connects the source-gas supplier and the liquefied gas reservoir; a sterilization filter provided at the supply pipe; a sterilizer that heats and sterilizes a sterilization region by high-temperature saturated water vapor, the sterilization region being located further downstream than the sterilization filter; and a dryer that removes moisture generated by the heat sterilization to dry the sterilization region.

## Description

### TECHNICAL FIELD

The present invention relates to a sterilized-liquefied gas producing apparatus, and particularly relates to a preferred technique used when a liquefied gas such as liquid nitrogen is produced and supplied under aseptic conditions.

This application claims priority from Japanese Patent Application No. 2016-125997 filed on June 24, 2016, the contents of which are incorporated herein by reference in their entirety.

### BACKGROUND ART

Liquefied gas such as liquid nitrogen in an aseptic state is increasingly used in the fields of medicine, pharmaceuticals, food, and research.

In accordance with this, an apparatus of producing a sterilized liquefied gas is required.

In Patent Documents 1 to 3, an apparatus is disclosed which includes a filter that sterilizes produced liquid nitrogen and sterilizes a pipe or the like that supplies liquid nitrogen.

Patent Document 1 proposes a method that sterilizes the inside of an apparatus filled with liquid nitrogen by heating by use of a high-temperature gas and maintains the sterility of the apparatus.

Patent Document 2 discloses that liquid nitrogen is sterilized by use of a filter made of a material having a resistance to extremely low temperature.

Patent Document 3 discloses that a sterilized gas is liquefied by cooling down and pipes or the like are sterilized by steam.

### PRIOR ART DOCUMENTS

### PATENT DOCUMENTS

[Patent Document 1] Japanese Unexamined Patent Application, First Publication No. 2000-185710
[Patent Document 2] Japanese Patent No. 4766226
[Patent Document 3] Published Japanese Translation No. 2013-531212 of PCT International Publication

### SUMMARY OF THE INVENTION

### PROBLEMS TO BE SOLVED BY THE INVENTION

However, according to the technique of Patent Document 1, a large amount of energy and time is required for heating the apparatus, and the apparatus must resist both ultralow temperatures due to liquid nitrogen and high temperatures due to a high-temperature gas.

Consequently, it is required that a wide temperature range such as approximately -200°C to +200°C as heat characteristics necessary for the components forming the apparatus, and there are problems in that a range to select materials used to form the components becomes narrow, the entire configuration of the apparatus becomes complicated, and the like.

Additionally, in Patent Document 2, a filter that can maintain a function of, for example, sterilizing liquid nitrogen for a long period of time is not present, and practically, it is not easy to produce aseptic liquid nitrogen.

Furthermore, Patent Document 3 discloses that a sterilized gas is liquefied by cooling down and the pipes or the like are sterilized by steam.

However, since a reservoir that stores liquefied gas is not provided, a supply amount of liquefied gas cannot be changed, moreover, there is a possibility that steam used for sterilization remains in the apparatus, and it is not possible to eliminate the possibility of bacteria breeding due to remaining moisture.

For this reason, there is problem in that it is not possible to guarantee that liquefied gas is in an aseptic condition.

The invention was conceived in view of the above-described circumstances and achieves an object to provide a sterilized-liquefied gas producing apparatus which is used for production and supply of a sterilized-liquefied gas, can continuously produce a sterilized-liquefied gas, and can guarantee that liquefied gas is in an aseptic condition.

### MEANS FOR SOLVING THE PROBLEMS

In order to solve the aforementioned problem, a sterilized-liquefied gas producing apparatus according to one aspect of the invention includes: a liquefied gas reservoir; a source-gas supplier that supplies a source gas to the liquefied gas reservoir; a cooler that cools down an inside of the liquefied gas reservoir to liquefy the source gas; a supply pipe that connects the source-gas supplier and the liquefied gas reservoir; a sterilization filter provided at the supply pipe; a sterilizer that heats and sterilizes a sterilization region by high-temperature saturated water vapor, the sterilization region being located further downstream than the sterilization filter; and a dryer that removes moisture generated by the heat sterilization to dry the sterilization region.

In the sterilized-liquefied gas producing apparatus according to one aspect of the invention, the dryer may supply a high-temperature inert gas to the sterilization region to carry out desiccation of the sterilization region.

In the sterilized-liquefied gas producing apparatus according to one aspect of the invention, the sterilizer and the dryer may be connected to the supply pipe that is located further upstream than the sterilization filter.

In the sterilized-liquefied gas producing apparatus according to one aspect of the invention, the supply pipe may be connected to an upper portion of the liquefied gas reservoir.

In the sterilized-liquefied gas producing apparatus according to one aspect of the invention, the cooler may include a freezing machine.

The sterilized-liquefied gas producing apparatus according to one aspect of the invention may further include: a reservoir recess that is capable of storing moisture generated by the heat sterilization and is provided at a bottom portion of the liquefied gas reservoir.

The sterilized-liquefied gas producing apparatus according to one aspect of the invention may further include: a discharge pipe that pressurizes an inside of the liquefied gas reservoir to discharge moisture generated by the heat sterilization and is provided at the liquefied gas reservoir.

The sterilized-liquefied gas producing apparatus according to one aspect of the invention may further include: a discharge pipe that pressurizes an inside of the liquefied gas reservoir to discharge moisture generated by the heat sterilization and is provided at the liquefied gas reservoir; and a reservoir recess that is capable of storing moisture generated by the heat sterilization and is provided at a bottom portion of the liquefied gas reservoir, wherein the discharge pipe penetrates through an upper portion of the liquefied gas reservoir, and an end of the discharge pipe is located close to the reservoir recess.

According to the sterilized-liquefied gas producing apparatus of one aspect of the invention, since the above-mentioned configuration is provided thereto, high-temperature saturated water vapor is supplied from the sterilizer to the sterilization filter serving as at least the sterilization region that is located further downstream than the sterilization filter, the supply pipe, and the inside of the liquefied gas reservoir.

Accordingly, the sterilization region is heated and sterilized, the moisture generated by the heat sterilization is removed by the dryer, the sterilization region is dried, and therefore sterilization treatment of the sterilization region is completed.

The source gas in an aseptic state is supplied filter from the source-gas supplier through the sterilization to the liquefied gas reservoir in the aseptic state, the cooler cools down the inside of the liquefied gas reservoir to liquefy the source gas, and the sterilized-liquefied gas is also stored in the liquefied gas reservoir.

Accordingly, it is possible to supply the sterilized-liquefied gas produced as needed to the outside.

Here, "aseptic" means a state where sterilization is achieved, and "sterilization" means that all of microorganisms and viruses that exist in a target object are annihilated or removed regardless of harmfulness or harmlessness.

Specifically, it means that sterility assurance level (SAL) is satisfied and means SAL≤10⁻⁶ (After the sterilization operation, the probability of microorganisms to survive in the object to be sterilized is less than 1 part per million).

Moreover, the liquefied gas is a gas having a normal boiling point of -50°C or less, and nitrogen, oxygen, liquid air and argon can be adopted.

In one aspect of the invention, the dryer supplies a high-temperature inert gas to the sterilization region to carry out desiccation of the sterilization region.

Consequently, after high-temperature saturated water vapor is supplied to the sterilization region by the sterilizer and sterilization treatment is thereby carried out, a high-temperature inert gas is supplied to the sterilization region by this dryer, and the moisture which is adhered to the sterilization filter serving as at least the sterilization region, the supply pipe, and the inside of the liquefied gas reservoir, or which is stored inside the liquefied gas reservoir is discharged to the outside.

As a result, regeneration of bacteria due to remaining moisture is prevented, improvement in sterilization of the sterilization region is achieved after sterilization treatment, it is possible to produce a sterilized-liquefied gas, and it is also possible to guarantee an aseptic state of the produced liquefied gas.

Here, nitrogen gas can be adopted as a high-temperature inert gas to be supplied to the sterilization region by the dryer.

Note that, as compared with a supply flow rate from the source-gas supplier when a liquefied gas is produced, a gas supply flow rate of a drying treatment is set preferably higher in terms of shortening an amount of time of drying treatment.

In one aspect of the invention, since the sterilizer and the dryer is connected to the supply pipe that is further upstream than the sterilization filter, the sterilizer and the dryer are in a state of being connected to the upstream side of the sterilization region.

Accordingly, since the entire of the sterilization region can be subjected to the sterilization treatment and the drying treatment, the entire of the sterilization region is in an aseptic state, production of a sterilized-liquefied gas can be carried out, and it is also possible to guarantee an aseptic state of the produced liquefied gas.

Additionally, in one aspect of the invention, since the supply pipe is connected to the upper portion of the liquefied gas reservoir, the supply pipe is not necessary to penetrate through the side surface and the bottom surface of the liquefied gas reservoir.

Because of this, a surface treatment on the inner surface of liquefied gas reservoir can be easily in a predetermined state.

Note that, in the sterilized-liquefied gas producing apparatus according to one aspect of the invention, the surface to be in contact with a liquefied gas or the inner surface of the sterilization region is in a state that satisfies a Sanitary Standard.

For this reason, it is possible to guarantee an aseptic state of the produced liquefied gas.

Here, Sanitary Standard means a standard used for production of foods, dairy husbandry, brewage, beverage, confectionery, seafood processing, medication, cosmetics, industrial chemical product, cold beverage, beer, alcohol, meat processing, chemical agent, semiconductor, or the like.

Furthermore, since the cooler includes the freezing machine, it is easy to cool down the inside of the liquefied gas reservoir to be a temperature at which a source gas is liquefied or a temperature less than the temperature, and it is possible to produce a large amount of liquefied gas which is stored inside the liquefied gas reservoir.

Moreover, the reservoir recess capable of storing the moisture generated by the heat sterilization is provided at the bottom portion of the liquefied gas reservoir.

By means of this structure, the moisture generated due to heat sterilization by the sterilizer is stored in the reservoir recess inside the liquefied gas reservoir, effectively discharged to the outside, and it is possible to easily maintain an aseptic state.

In one aspect of the invention, the discharge pipe that pressurizes an inside of the liquefied gas reservoir to discharge the moisture after the heat sterilization is provided at the liquefied gas reservoir.

As a result, only by applying a pressure to the liquefied gas reservoir, the moisture generated due to heat sterilization by the sterilizer is effectively discharged from the inside of the liquefied gas reservoir to the outside through the discharge pipe, the moisture of the sterilization region is eliminated, regeneration of bacteria is prevented, and it is possible to easily maintain an aseptic state.

In the sterilized-liquefied gas producing apparatus according to one aspect of the invention, the discharge pipe that pressurizes an inside of the liquefied gas reservoir to discharge the moisture after the heat sterilization is provided at the liquefied gas reservoir, the reservoir recess capable of storing the moisture generated by the heat sterilization is provided at the bottom portion of the liquefied gas reservoir, the discharge pipe penetrates through the upper portion of the liquefied gas reservoir, and the end of the discharge pipe is located close to the reservoir recess.

By employing the above-described configuration, the moisture generated due to heat sterilization by the sterilizer is stored in the reservoir recess inside the liquefied gas reservoir, the moisture stored in the reservoir recess is effectively discharged from the inside of the liquefied gas reservoir to the outside through the discharge pipe only by applying a pressure to the liquefied gas reservoir, the moisture of the sterilization region is eliminated, regeneration of bacteria is prevented, and it is possible to easily maintain an aseptic state.

### Effects of the Invention

According to one aspect, in the sterilization region in which the liquefied gas is produced and stored, sterilization is carried out by high-temperature saturated water vapor, and moisture is removed by the dryer.

Consequently, the effect is obtained that it is possible to provide the sterilized-liquefied gas producing apparatus that prevents regeneration of bacteria in the sterilization region, easily maintains an aseptic state, can carry out production of a sterilized-liquefied gas, and can guarantee an aseptic state of the produced liquefied gas.

### BRIEF DESCRIPTION OF THE DRAWINGS

FIG. 1 is a schematic diagram showing a sterilized-liquefied gas producing apparatus according to a first embodiment of the invention.
FIG. 2 is a flowchart showing steps of producing a liquefied gas in the sterilized-liquefied gas producing apparatus according to the first embodiment of the invention.

### EMBODIMENTS FOR CARRYING OUT THE INVENTION

Hereinafter, a sterilized-liquefied gas producing apparatus according to a first embodiment of the invention will be described with reference to drawings.

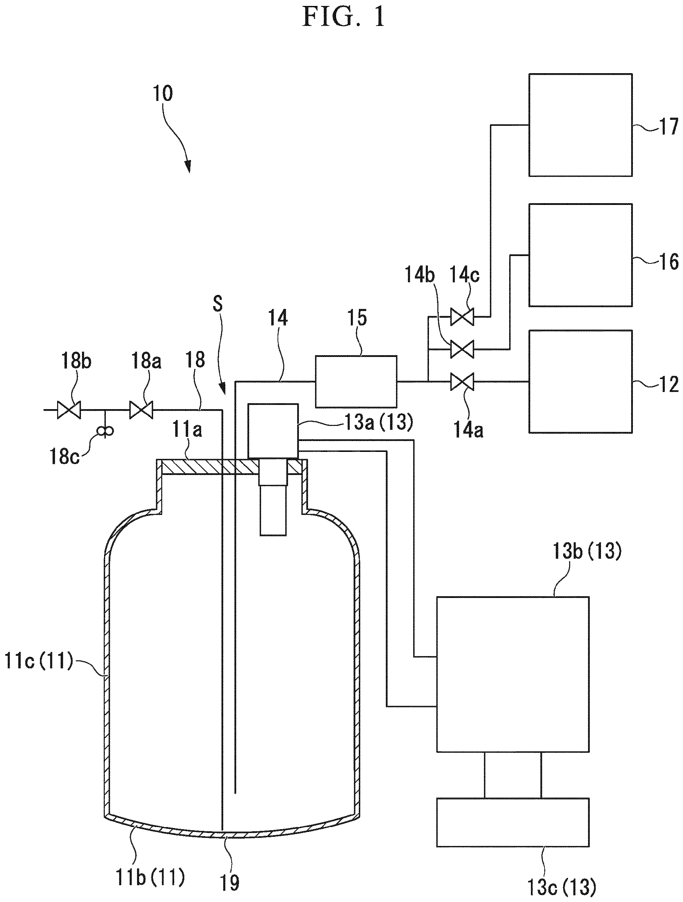

FIG. 1 is a schematic cross-sectional diagram showing a sterilized-liquefied gas producing apparatus according to the first embodiment of the invention, reference numeral 10 in this diagram represents a sterilized-liquefied gas producing apparatus.

As shown in FIG. 1, the sterilized-liquefied gas producing apparatus 10 according to the embodiment includes a liquefied gas reservoir 11, a source-gas supplier 12, a cooler 13, a supply pipe 14, a sterilization filter 15, a sterilizer 16, a dryer 17, and discharge pipe 18.

The liquefied gas reservoir 11 according to the embodiment is a hermetically-sealed reservoir as shown in FIG. 1 which is formed in a bottomed and substantially cylindrical shape, and satisfies sanitary specification which will be described later.

As shown in FIG. 1, the supply pipe 14, the discharge pipe 18, and the cooler 13 are provided at the liquefied gas reservoir 11 so as to penetrate through a lid 11a serving as an upper end thereof.

A slope that smoothly lowers from the connection position between the bottom portion 11b and the side wall 11c toward the center of the bottom portion 11b is formed on a bottom portion 11b of the liquefied gas reservoir 11.

A recess is formed at the central of the bottom portion 11b, and the recess forms a reservoir recess 19.

Particularly, the bottom portion 11b is formed in a curved surface shape such that a spherical surface is cut off, the strength of the liquefied gas reservoir 11 can be maintained in the case where a difference in pressure between the liquefied gas reservoir 11 and the outside occurs.

The source-gas supplier 12 is configured to supply a source gas serving as a raw material used to form a liquefied gas to the liquefied gas reservoir 11 and is connected to the supply pipe 14 via a valve 14a.

In the embodiment, the source-gas supplier 12 supplies nitrogen gas to the liquefied gas reservoir 11 as a source gas.

The source-gas supplier 12 supplies a source gas having, for example, substantially a room temperature, to the liquefied gas reservoir 11.

The source-gas supplier 12 according to the embodiment includes a nitrogen generator using an absorbing agent (PSA), effectively separate air into oxygen and nitrogen, and can supply up to 99.99% of nitrogen gas to the liquefied gas reservoir 11.

In other cases, as long as a source gas can be supplied to the liquefied gas reservoir 11, the configuration of the source-gas supplier 12 is not limited to the above-mentioned configuration.

As shown in FIG. 1, the cooler 13 is a freezing machine system and is configured to include: a cooling unit 13a that functions as a mechanical freezing machine and penetrates through the lid 11a serving as an upper end of the liquefied gas reservoir 11; a compressor 13b that is connected to the cooling unit 13a and is used to supply and retrieve a refrigerant gas; and a water cooling unit 13c connected to the compressor 13b.

The cooling unit 13a penetrates through the lid 11a and protrudes toward the inside of the liquefied gas reservoir 11, and is configured to liquefy the source gas due to heat exchange by the cooling unit 13a that functions as a mechanical freezing machine.

The cooling unit 13a can cool down a cooling medium (for example, helium gas) supplied from the compressor 13b until being a ultralow temperature of, for example, 80K by causing the cooling medium to be subjected to Simon expansion.

The compressor 13b is configured to circulate, for example, helium gas between the cooling unit 13a and the compressor 13 and remove heat from the cooling unit 13a.

The heat is discharged to by the water cooling unit 13c.

In other cases, a configuration (air cooling) may be adopted which directly discharges heat by the compressor 13b without providing the water cooling unit 13c.

The cooling unit 13a that protrudes into the inside of the liquefied gas reservoir 11 is configured so that the surface thereof satisfies sanitary specification.

The downstream side of the supply pipe 14 penetrates through the lid 11a serving as an upper end of the liquefied gas reservoir 11 and is located in an axial direction at the center of the liquefied gas reservoir 11, and the supply pipe 14 is provided so as to longitudinally extend into the inside of the liquefied gas reservoir 11.

A plurality of through holes are provided along the entire length of the pipe side surface of the supply pipe 14 that extends into the inside of the liquefied gas reservoir 11, and the inside of the supply pipe 14 that extends into the inside of the liquefied gas reservoir 11 is communicated with the inside of the liquefied gas reservoir 11.

The sterilization filter 15 is provided on the upstream side of the supply pipe 14.

Additionally, the pipe located at the upstream side of the sterilization filter 15 is formed so as to split into three pipes.

The split pipes are connected to the source-gas supplier 12, the sterilizer 16, and the dryer 17 via valves 14a, 14b, and 14c, respectively.

In other cases, the valve 14a, the valve 14b, the valve 14c may be formed by three independent valves; however, as long as the structure that can switch three branches is adopted, another configuration may be adopted.

The sterilization filter 15 is provided at the supply pipe 14 and is a filter that can sterilize a source gas supplied from the source-gas supplier 12.

The filter performance of the sterilization filter 15 can exhibits around a room temperature.

Moreover, as described below, the sterilization filter 15 is formed by a material and in a shape which does not degrade the filter performance by high-temperature saturated water vapor supplied from the sterilizer 16.

In order to cause a liquefied gas which is to be produced to be in an aseptic state, the sterilization filter 15, the supply pipe 14 positioned further downstream than the sterilization filter 15, the inside of the liquefied gas reservoir 11, and the discharge pipe 18 are regarded as a sterilization region S that maintains a state where sterilization is achieved when a liquefied gas is produced.

As the sterilization filter 15, for example, a hydrophobic PTFE membrane filter used for gas sterilization, produced by PALL Corporation, can be adopted.

The sterilization filter 15 is configured such that a plurality of fluidic channels are replaceably provided in the inside of the sterilization filter 15, and for example, is configured to be able to replace two or three filters in parallel.

The sterilizer 16 includes a saturated water vapor generator capable of supplying high-temperature saturated water vapor set to greater than or equal to, for example, 121°C, and is connected to the supply pipe 14 branched via the valve 14b.

When the valve 14b is opened, the valve 14a and the valve 14c are closed, and supply of high-temperature saturated water vapor is carried out by the sterilizer 16, it is possible to heat sterilize the sterilization region S that is further downstream than the sterilization filter 15.

The dryer 17 is configured to supply a high-temperature inert gas greater than or equal to 100°C to the sterilization region S, thereby remove the moisture generated due to the heat sterilization, and dry the inside of the sterilization region S.

As an inert gas that is to be supplied to the sterilization region S, for example, nitrogen gas can be adopted.

Note that, when drying is carried out, it is preferable that nitrogen gas can be supplied which has a flow rate higher than a supply amount of the source gas used to form a liquefied gas.

The upstream side of the discharge pipe 18 penetrates through the lid 11a serving as an upper end of the liquefied gas reservoir 11 and is located in an axial direction at the center of the liquefied gas reservoir 11, and the discharge pipe 18 is provided so as to longitudinally extend into the inside of the liquefied gas reservoir 11.

The edge of the discharge pipe 18 on the upstream side is positioned so as to substantially come into contact with the central region which is located at the lowest position of the reservoir recess 19.

The discharge pipe 18 is configured as a drain capable of discharging the moisture (hot liquid), which is generated by the heat sterilization and accumulated in the reservoir recess 19, to the outside.

On the downstream side of the discharge pipe 18, a valve 18a is provided at the position which is outside the liquefied gas reservoir 11, and a valve 18b is provided at the downstream side of the valve 18a.

An output port 18c that supplies a liquefied gas to the outside is provided between the valve 18a and the valve 18b so as to separate from the valve 18b.

On the downstream side of the valve 18b, a cooler may be provided which is not shown in the figure and cools down vapor discharged from the valve 18b when high-temperature saturated water vapor is supplied from the sterilizer 16.

The output port 18c is configured to be openable and closable, may store the liquefied gas that is extracted from the output port 18c to the outside while being unmodified. Alternatively, the output port 18c is directly connected through a pipe to the region or the like which uses a liquefied gas in an aseptic state in the fields of medicine, pharmaceuticals, food, research, or the like, and may supply a liquefied gas to another apparatus or the like while being unmodified.

A pressure detecting device that measures an internal pressure thereof, a temperature detecting device that measures an internal temperature thereof, a heater that heats the inside thereof when heat sterilization is carried out, a diaphragm that applies a pressure thereof, a safety valve that operates in the case where the internal pressure thereof increases greater than or equal to a predetermined value, or the like, which are not shown in the figure, are provided on the liquefied gas reservoir 11.

Of the above-mentioned devices (pressure detecting device, temperature detecting device, heater, diaphragm, safety valve), the device that needs to be communicated with the outside of the liquefied gas reservoir 11 is arranged so as to penetrate through the lid 11a serving as an upper end of the liquefied gas reservoir 11.

In other cases, a plurality of the temperature detecting devices can be provided inside the liquefied gas reservoir 11.

As a position at which the temperature detecting device is provided, for example, the upper position on the side wall of the liquefied gas reservoir 11, the position which is near the cooling unit 13a and at which a temperature of the cooling unit 13a can be detected, the position which is the lower end of the liquefied gas reservoir 11 and is near the bottom portion 11b, the position near the heater that heats the inside of the liquefied gas reservoir 11, or the like is adopted.

In the sterilized-liquefied gas producing apparatus 10 according to the embodiment, the supply pipe 14, the sterilization filter 15, the liquefied gas reservoir 11, the cooling unit 13a, the discharge pipe 18, the lower end of the discharge pipe 18 which is located close to the valve 18b, and the output port 18c, which function as the sterilization region S are configured so that the surfaces thereof or the inner surfaces thereof satisfy sanitary specifications.

As a material that satisfies sanitary specification, for example, stainless steels, particularly, SUS316 or SUS316L is adopted.

Furthermore, by carrying out a specular finishing treatment by use of an abrasive of No. 400 and an electrochemical polishing treatment with respect to the surface of such stainless steels, it is possible to achieve the specification in compliance with a stainless steel sanitary pipe or the like which is determined by JIS standards.

Alternatively, it is also possible to use the configuration in which the surface of the aforementioned stainless steels or the surface which was subjected to the aforementioned treatment is coated with Au, Pt, or the like.

Moreover, components, joints of pipes, O-rings provided on joints, or the like which form the above-described sterilization region S can be made of silicone satisfying Sanitary Standard, fluorine resin, fluorine rubber such as vinylidene fluoride (FKM), or the like.

Hereinafter, a method of producing a sterilized-liquefied gas in the sterilized-liquefied gas producing apparatus according to the embodiment will be described.

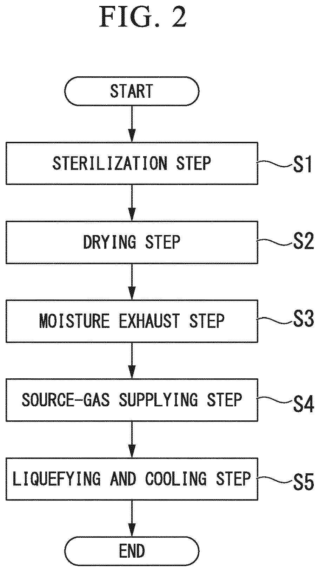

FIG. 2 is a flowchart showing steps of producing a liquefied gas in the sterilized-liquefied gas producing apparatus according to the embodiment.

As shown in FIG. 2, the method of producing a sterilized-liquefied gas in the sterilized-liquefied gas producing apparatus 10 according to the embodiment includes: a sterilization step S1, a drying step S2, a moisture exhaust step S3, a source-gas supplying step S4, and a liquefying and cooling step S5.

In the method of producing a sterilized-liquefied gas in the sterilized-liquefied gas producing apparatus 10 according to the embodiment, as shown in FIG. 2, firstly, the sterilization region S is sterilized in the sterilization step S1.

In the sterilization step S1, firstly, the valve 14a and the valve 14c are in a closed state, the valve 14b is in an open state, the cooler 13 is in a stopped state, the source-gas supplier 12 is in a stopped state, the valve 18a is in an open state, the valve 18b is in an open state, and the output port 18c is in an open state.

In this state, the sterilizer 16 is operated, and high-temperature saturated water vapor which is generated by the saturated water vapor generator and is set to greater than or equal to 121°C is supplied to the supply pipe 14 through the valve 14b.

The high-temperature saturated water vapor passes through the valve 14b, passes through the supply pipe 14, the sterilization filter 15, the liquefied gas reservoir 11, and the discharge pipe 18 which are the sterilization region S, and is discharged from the lower end of the discharge pipe 18 which is located near the valve 18b and the output port 18c.

Accordingly, the high-temperature saturated water vapor comes into contact with the inner surfaces of the components forming the sterilization region S, sterilization treatment is carried out on the inner surfaces of the components forming the sterilization region S, with which the high-temperature saturated water vapor was in contact, and bacteria is annihilated from the inner surfaces of the sterilization region S.

At this time, the inside of the liquefied gas reservoir 11 may be heated by a heater which is not shown in the figure or a pressure may be applied to the inside of the liquefied gas reservoir 11 by a diaphragm which is not shown in the figure.

As treatment conditions in the sterilization step S1, in order to completely obtain an aseptic state, the sterilization conditions can be adopted which are required for, for example, a freezing dryer used for medicinal products.

In such treatment conditions, the sterilization region S is subjected to, for example, steam having 121°C or more, for 20 minutes or more, for example, approximately 30 minutes, and bacteria is annihilated by maintaining this temperature condition.

A pressure of the high-temperature saturated water vapor in the sterilization step S1 may be approximately 210 kPa, approximately 220 kPa to 240 kPa.

At this time, on the inner surfaces of the components forming the sterilization region S with which the high-temperature saturated water vapor was in contact, the high-temperature saturated water vapor becomes high-temperature moisture (hot water), the moisture (hot water) generated by heat sterilization is discharged to the outside.

Furthermore, the high-temperature moisture (hot water) generated from the high-temperature saturated water vapor is stored the reservoir recess 19 of the bottom portion 11b of the liquefied gas reservoir 11 in the supply pipe 14, the sterilization filter 15, the liquefied gas reservoir 11.

In this case, since the upper end of the discharge pipe 18 is provided so as to be in contact with the bottom portion 11b serving as the reservoir recess 19, due to the principle of siphon, the high-temperature moisture (hot water) is promptly discharged from the lower end of the discharge pipe 18 which is located close to the valve 18b and the output port 18c in the discharge pipe 18.

After it is observed that the sterilization region S is maintained in the steam in a high-temperature state for a predetermined amount of time, the operation of the sterilizer 16 is stopped, and the sterilization step S1 is completed.

Next, in the drying step S2 shown in FIG. 2, the valve 14b is in a closed state, the valve 14c is in an open state.

In this state, the dryer 17 is operated, and a high-temperature inert gas greater than or equal to 100°C is supplied to the supply pipe 14.

For example, a high-temperature inert gas that is nitrogen gas passes through the valve 14c, passes through the supply pipe 14, the sterilization filter 15, the liquefied gas reservoir 11, and the discharge pipe 18 which are the sterilization region S, and is discharged from the lower end of the discharge pipe 18 which is located close to the valve 18b and the output port 18c to the outside.

Consequently, the moisture (hot water) generated by heat sterilization in the sterilization step S1 is removed and the inside of the sterilization region S is dried.

At this time, the inside of the liquefied gas reservoir 11 may be heated by a heater which is not shown in the figure or a pressure may be applied to the inside of the liquefied gas reservoir 11 by a diaphragm which is not shown in the figure.

In the drying step S2, since it is necessary to dry the inside of the sterilization region S such as the liquefied gas reservoir 11, a high-temperature inert gas having a flow rate higher than a supply amount of the source gas from the source-gas supplier 12 in the liquefying and cooling step S5 which will be described later is supplied from the dryer 17 to the inside of the sterilization region S.

The high-temperature inert gas supplied from the dryer 17 passes through the sterilization filter 15 in an aseptic state and is therefore supplied to the inside of the sterilization region S in an aseptic state where bacteria is annihilated.

Subsequently, in the moisture exhaust step S3 shown in FIG. 2, continuous to the drying step S2, a pressure is applied to the inside of the liquefied gas reservoir 11 by the high-temperature inert gas supplied from the dryer 17.

At this time, the inside of the liquefied gas reservoir 11 may be heated by a heater which is not shown in the figure or a pressure may be applied to the inside of the liquefied gas reservoir 11 by a diaphragm which is not shown in the figure.

After the moisture (hot water) which is generated by heat sterilization and is accumulated in the reservoir recess 19 is discharged to the outside, the moisture adhered to the inner surfaces of the sterilization region S evaporates due to the high-temperature inert gas supplied from the dryer 17 and is discharged from the discharge pipe 18.

After it is observed that the moisture adhered to not only the inside of the liquefied gas reservoir 11 but also the inner surfaces of the sterilization region S is completely discharged to the outside, the operation of the dryer 17 is stopped, and the moisture exhaust step S3 is completed.

Note that, the drying step S2 and the moisture exhaust step S3 were described as independent step for convenience in the embodiment; however, the steps can be simultaneously carried out.

After that, in the source-gas supplying step S4 shown in FIG. 2, the valve 14c and the valve 18b are in a closed state, the valve 14a is in an open state, and the output port 18c is in an open state.

In this state, the source-gas supplier 12 is operated, and nitrogen gas serving as a source gas is supplied to the liquefied gas reservoir 11.

At this time, the source gas supplied from the source-gas supplier 12 flows in the supply pipe 14 through the sterilization filter 15 into the liquefied gas reservoir 11; however, the region into which the source gas flows is the sterilization region S that is located further downstream than the sterilization filter 15.

Since the sterilization treatment by the sterilizer 16 is completely finished, the portion serving as the sterilization region S located further downstream than the sterilization filter 15 can be maintained in an aseptic state.

Next, in the liquefying and cooling step S5 shown in FIG. 2, the cooler 13 is operated, the compressor 13b circulates helium gas between the compressor 13b and the cooling unit 13a, removes heat from the cooling unit 13a, and the heat is discharged to the outside by the water cooling unit 13c.

Consequently, as the cooling unit 13a is cooled down which penetrates through the lid 11a and protrudes toward the inside of the liquefied gas reservoir 11, the inside of the liquefied gas reservoir 11 is cooled down, and the source gas is liquefied.

The liquefied source gas is stored inside the liquefied gas reservoir 11.

As necessary, the sterilized-liquefied gas stored in the liquefied gas reservoir 11 can be supplied from the output port 18c to the outside by applying a pressure to the inside of the liquefied gas reservoir 11.

Here, when the source gas is supplied to the liquefied gas reservoir 11 while maintaining the output port 18c in an open state, in the drying step S2, the internal pressure of the liquefied gas reservoir 11 is higher than the atmospheric pressure (1 atm + α) and is in a state where the temperature thereof is also higher.

The reason for this is that, the cooler 13 is not promptly cooled down even being operated, the internal pressure of the liquefied gas reservoir 11 increases until being the supply pressure of the source-gas supplier 12.

At the same time, in order to prevent bacteria from entering to the liquefied gas reservoir by setting the gas flow to be directed in a single direction, supply of the source gas by the source-gas supplier 12 is adjusted so that the inside pressure of the liquefied gas reservoir 11 is atmospheric pressure + α.

Additionally, when the source gas is supplied and liquefying operation is carried out, the output port 18c is maintained in an open state.

Furthermore, in the case where liquid is stored inside the liquefied gas reservoir 11 without supplying the source gas, the pressure inside the liquefied gas reservoir 11 increases by evaporation of the source gas due to heat input.

For this reason, by driving the pressure detecting device which is not shown in the figure simultaneously with the valve 18b, the internal pressure of the liquefied gas reservoir 11 is controlled to be low until being a setting value.

Additionally, normally, the safety valve is not operated.

In the production of the sterilized-liquefied gas in the sterilized-liquefied gas producing apparatus 10 according to the embodiment, the sterilization region S is in an aseptic state by the sterilization step S1, the drying step S2, and the moisture exhaust step S3.

Since this aseptic state is maintained and the treatment of liquefying the source gas can be carried out, it is possible to produce a sterilized-liquefied gas.

Furthermore, as stated above, since the aseptic state is maintained, it is possible to guarantee an aseptic state of the produced sterilized-liquefied gas.

In the embodiment, the sterilization filter 15 is in an aseptic state by the sterilization step S1, the drying step S2, and the moisture exhaust step S3.

In this state, when the source gas is supplied from the source-gas supplier 12 toward the liquefied gas reservoir 11 through the supply pipe 14, the source gas passes through the sterilization filter 15.

The source gas that becomes in an aseptic state by the sterilization filter 15 is supplied to the inside of the liquefied gas reservoir 11, and a liquefying process can be carried out.

In the embodiment, since the reservoir recess 19 is provided, water is collected along the inclined surface (angle) formed on the bottom surface of the bottom portion 11b of the liquefied gas reservoir 11, and it is possible to discharge the accumulated water (hot water).

In the embodiment, since the discharge pipe 18 is formed in a siphon shape, the hot water accumulated in the reservoir recess 19 can be completely discharged to the outside, and therefore the inside of the liquefied gas reservoir 11 and the discharge pipe 18 can be in an aseptic state.

Moreover, since the discharge pipe 18 is arranged so as to come into contact with the reservoir recess 19, even in cases where extraneous materials exist in the produced liquefied gas, the extraneous materials can be discharged to the outside if the extraneous materials are in a state of being accumulated in the reservoir recess 19.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

### Industrial Applicability

As application examples of the invention, provision of aseptic liquid nitrogen can be adopted which can be directly used for aseptic medicines or the like in the field of bio medicine and regenerative medicine or in the case where it is necessary to rapidly cool down a product in a freezing dryer or which can be directly used in an aseptic room.

### DESCRIPTION OF REFERENCE NUMERAL

10...sterilized-liquefied gas producing apparatus
11...liquefied gas reservoir
12...source-gas supplier
13... cooler (mechanical freezing machine system)
13a...cooling unit
13b...compressor
13c...water cooling unit
14...supply pipe
14a, 14b, 14c...valve
15...sterilization filter
16...sterilizer
17...dryer
18...discharge pipe
18a, 18b...valve
18c...output port
19...reservoir recess
S...sterilization region

## Claims

1. A sterilized-liquefied gas producing apparatus, comprising:
a liquefied gas reservoir;
a source-gas supplier that supplies a source gas to the liquefied gas reservoir;
a cooler that cools down an inside of the liquefied gas reservoir to liquefy the source gas;
a supply pipe that connects the source-gas supplier and the liquefied gas reservoir;
a sterilization filter provided at the supply pipe;
a sterilizer that heats and sterilizes a sterilization region by high-temperature saturated water vapor, the sterilization region being located further downstream than the sterilization filter; and
a dryer that removes moisture generated by the heat sterilization to dry the sterilization region.

2. The sterilized-liquefied gas producing apparatus according to claim 1, wherein
the dryer supplies a high-temperature inert gas to the sterilization region to carry out desiccation of the sterilization region.

3. The sterilized-liquefied gas producing apparatus according to claim 1 or claim 2, wherein
the sterilizer and the dryer are connected to the supply pipe that is located further upstream than the sterilization filter.

4. The sterilized-liquefied gas producing apparatus according to any one of claims 1 to 3, wherein
the supply pipe is connected to an upper portion of the liquefied gas reservoir.

5. The sterilized-liquefied gas producing apparatus according to any one of claims 1 to 4, wherein
the cooler includes a freezing machine.

6. The sterilized-liquefied gas producing apparatus according to any one of claims 1 to 5, further comprising:
a reservoir recess that is capable of storing moisture generated by the heat sterilization and is provided at a bottom portion of the liquefied gas reservoir.

7. The sterilized-liquefied gas producing apparatus according to any one of claims 1 to 6, further comprising:
a discharge pipe that pressurizes an inside of the liquefied gas reservoir to discharge moisture generated by the heat sterilization and is provided at the liquefied gas reservoir.

8. The sterilized-liquefied gas producing apparatus according to any one of claims 1 to 5, further comprising:
a discharge pipe that pressurizes an inside of the liquefied gas reservoir to discharge moisture generated by the heat sterilization and is provided at the liquefied gas reservoir; and
a reservoir recess that is capable of storing moisture generated by the heat sterilization and is provided at a bottom portion of the liquefied gas reservoir, wherein
the discharge pipe penetrates through an upper portion of the liquefied gas reservoir, and an end of the discharge pipe is located close to the reservoir recess.
